# EUROPEAN PATENT SPECIFICATION

(11) **EP 0 728 194 B1**
(45) Date of publication and mention of the grant of the patent: **08.01.2003**
(21) Application number: 94931482.7
(22) Date of filing: 08.11.1994
(51) Int. Cl.: C12N 5/06, A61K 38/18, A61K 48/00

(54) **IN SITU MODIFICATION AND MANIPULATION OF STEM CELLS OF THE CENTRAL NERVOUS SYSTEM**
IN SITU MODIFIKATION UND MANIPULATION VON STAMMZELLEN DES ZENTRALEN NERVENSYSTEMS
MODIFICATION ET MANIPULATION IN SITU DE CELLULES SOUCHES DU SYSTEME NERVEUX CENTRAL

(30) Priority: 09.11.1993 US 149508
(43) Date of publication of application: 28.08.1996
(73) Proprietor: NEUROSPHERES HOLDINGS LTD., Calgary, Alberta T2N 4N1 (CA)
(72) Inventor: WEISS, Samuel, Calgary Alberta T2L 1A6 (CA); REYNOLDS, Brent, A., Calgary,Alberta T3T 2N2 (CA); VAN DER KOOY, Derek, Toronto Ontario M5S 1A8 (CA); MORSHEAD, Cindi, Toronto Ontario M6S 3T9 (CA); CRAIG, Constance, Toronto Ontario M5S 1A8 (CA)
(74) Representative: Barth, Renate, Dr.
(86) International application number: CA9400614
(87) International publication number: WO95013364

(56) References cited:
- EP-A- 0 312 208
- EP-A- 0 454 026
- EP-A- 0 455 422
- WO-A-90/06757
- WO-A-93/08825
- WO-A-94/16718
- US-A- 5 034 375
- SCIENCE, vol.255, no.5052, 27 March 1992, WASHINGTON DC, USA pages 1707 - 1710 B. REYNOLDS ET AL. 'Generation of neurons and astrocytes from isolated cells of the adult mammalian central nervous system.'
- ANNALS OF THE NEW YORK ACADEMY OF SCIENCES, vol.692, 27 August 1993, NEW YORK NY, USA pages 321 - 334 F. MCMORRIS ET AL. 'Regulation of oligodendrocyte development and central nervous system myelination by insulin-like growth factors.'
- NEURON, vol.11, no.5, November 1993, CAMBRIDGE MA, USA pages 951 - 966 A. VESCOVI ET AL. 'bFGF regulates the proliferative fate of unipotent (neuronal) and bipotent (neuronal/astroglial) EGF-generated CNS progenitor cells.'
- PROCEEDINGS OF THE NATIONAL ACADEMY OF SCIENCES OF THE USA, vol.84, no.1, January 1987, WASHINGTON DC, USA pages 156 - 160 J. PRICE ET AL. 'Lineage analysis in the vertebrate nervous system by retrovirus-mediated gene transfer.'
- NATURE GENETICS, vol.3, no.3, March 1993, NEW YORK NY, USA pages 219 - 223 B. DAVIDSON ET AL. 'A model system for in vivo gene transfer into the central nervous system using an adenoviral vector.'

## Description

The present invention is directed to the *in situ* (*in vivo*) modification and manipulation, via genetic or epigenetic means, of endogenous precursor cells in the mammalian central nervous system (CNS) and compositions for inducing *in situ* modification and manipulation of CNS precursor cells. More particularly, the invention is related to a use of a combination comprising epidermal growth factor and fibroblast growth factor in the manufacture of a pharmaceutical composition for treatment of neurological disease, disorder, and/or injury.

Development of the mammalian nervous system begins in the early stage of fetal development and continues until the early post-natal period. The mammalian central nervous system (CNS) is composed of three cell types: neurons, astrocytes and oligodendrocytes. While neurons are generated primarily during the fetal period, oligodendrocytes and astrocytes are generated during the early post-natal period. By the late post-natal period the CNS has its full complement of nerve cells.

Unlike many other cells found in different tissues, the differentiated cells of the adult mammalian CNS have little or no ability to enter the mitotic cycle and generate new nerve cells. While it is believed that there is a limited and slow turnover of astrocytes [Korr et al., *J. Comp. Neurol 150*:169, (1971)] and that progenitors for oligodendrocytes [Wolsqijk and Noble, *Development 105*:386, (1989)] are present, the generation of new neurons does not occur. While a few mammalian species (e.g. rats) exhibit the limited ability to generate new neurons in restricted adult brain regions such as the dentate gyrus and olfactory bulb [Kaplan, *J. Comp. Neurol*. *195*:323, (1981); Bayer, *N.Y. Acad. Sci. 457*:163, (1985]) this does not apply to all mammals; and the generation of new CNS cells in adult primates does not occur [Rakic, *Science* 227:1054, (1985)]. This inability to produce new nerve cells in most mammals (and especially primates) may be advantageous for long-term memory retention; however, it is a distinct disadvantage when the need to replace lost neuronal cells arises due to injury or disease.

CNS disorders encompass numerous afflictions such as neurodegenerative diseases (e.g. Alzheimer's and Parkinson's), acute brain injury (e.g. stroke, head injury, cerebral palsy) and a large number of CNS dysfunctions (e.g. depression, epilepsy, and schizophrenia). In recent years neurodegenerative disease has become an important concern due to the expanding elderly population which is at greatest risk for these disorders. These diseases, which include Alzheimer's Disease, Multiple Sclerosis, Huntington's Disease, Amyotrophic Lateral Sclerosis, and Parkinson's Disease, have been linked to the degeneration of neural cells in particular locations of the CNS, leading to the inability of these cells or the brain region to carry out their intended function. In addition to neurodegenerative diseases, acute brain injuries often result in the loss of neural cells, the inappropriate functioning of the affected brain region, and subsequent behavior abnormalities. Probably the largest area of CNS dysfunction (with respect to the number of affected people) is not characterized by a loss of neural cells but rather by an abnormal functioning of existing neural cells. This may be due to inappropriate firing of neurons, or the abnormal synthesis, release, and processing of neurotransmitters. Some of these dysfunctions are well studied and characterized disorders such as depression and epilepsy, others are less understood disorders such as neurosis and psychosis.

To date, treatment for CNS disorders has been primarily via the administration of pharmaceutical compounds. Unfortunately, this type of treatment has been fraught with many complications including the limited ability to transport drugs across the blood-brain barrier and the drug-tolerance which is acquired by patients to whom these drugs are administered long-term. For instance, partial restoration of dopaminergic activity in Parkinson's patients has been achieved with levodopa, which is a dopamine precursor able to cross the blood-brain barrier. However, patients become tolerant to the effects of levodopa, and therefore, steadily increasing dosages are needed to maintain its effects. In addition, there are a number of side effects associated with levodopa such as increased and uncontrollable movement.

Recently, the concept of neurological tissue grafting has been applied to the treatment of neurodegenerative diseases such as Parkinson's Disease.

An emerging technology for treating neurological disorders entails the transplantation of cells into the CNS to replace or compensate for loss or abnormal functioning of the host's nerve cells. While embryonic CNS cells have given the best results in human trials [Winder *et al., New Eng. J. Med. 327*:1556, (1992)] and are the preferred donor tissue, due to ethical and political considerations which limit the use of these cells, other types of donor tissue are being developed. These include: genetically modified neural cell lines [Renfranz *et al., Cell 66*:173, (1991); Synder *et al., Cell 68*:1, (1992)], fibroblasts [Kawaja *et al., J. Neurosci., 12*:2849, (1992)], muscle cells [Jiao *et al., Nature 363*:456, (1993)], glial progenitor cells [Groves *et al., Nature 362*:453, (1993)] and encapsulated cells [Hoffman *et al., Exp. Neurol. 132*:100, (1993)].

While transplantation approaches represent a significant improvement over currently available treatments for neurological disorders, they suffer from a number of significant drawbacks. For example, upon transplantation, some cell types fail to integrate with host tissue. Another disadvantage is that immunological incompatibility between donor and host could result in the rejection of the implanted cells. There is also the potential that the transplanted cells can result in tumor formation or pass infectious agents from the donor tissue to the host. Another significant drawback of transplantation procedures is that due to the invasiveness of the procedures. which carry the risks involved in any major neurosurgical operation, damage to healthy brain tissue could occur.

Leblond [*NCI Monograph, 14*:19, (1963)] has classified adult mammalian tissue into three categories based on the rate of cell turnover within the population. These are:
1. Static populations, like the nervous system, where there is little or no division and cells formed during development persist for the animal's lifetime;
2. Tissues having a conditionally renewing population of cells, such as the liver, where there is little cell division under normal conditions but where new cells can generate in response to an appropriate stimulus;
3. Continually renewing populations, such as the blood and squamous epithelium, where differentiated cells have a short life span and are continually being replaced by a subpopulation of cells referred to as stem cells.

The critical identifying feature of a stem cell is its ability to exhibit self-renewal or to generate more of itself. The simplest definition of a stem cell would be a cell with the capacity for self-maintenance. A more stringent (but still simplistic) definition of a stem cell is provided by Potten and Loeffler [*Development 110*:1001, (1990)] who have defined stem cells as "undifferentiated cells capable of a) proliferation, b) self-maintenance, c) the production of a large number of differentiated functional progeny, d) regenerating the tissue after injury, and e) a flexibility in the use of these options."

The role of stem cells is to replace cells that are lost by natural cell death, injury or disease. The presence of stem cells in a particular type of tissue usually correlates with tissues that have a high turnover of cells. However, this correlation may not always hold as stem cells are thought to be present in tissues that do not have a high turnover of cells [e.g., liver; Travis, *Science, 259*:1829, (1993)].

The low turnover of cells in the mammalian CNS together with the inability of the adult mammalian CNS to generate new cells in response to the loss of cells following injury or disease has lead to the assumption that the adult mammalian CNS does not contain stem cells. However, cells exhibiting stem cell characteristics *in vitro* have recently been isolated from the CNS. This cell is present in the embryo [Reynolds *et al., J. Neurosci. 12*:4565, (1992)] through to the adult [Reynolds and Weiss, *Science 255*:1707, (1992)], suggesting that adult CNS, although it does not generate new cells in response to injury or disease, has the capacity to generate new cells and to repair itself via proliferation and differentiation of stem cells and their progeny in a manner analogous to the hematopoetic system.

In consideration of the limitation and deficiencies attendant in the prior art with respect to therapies for treating CNS disorders, it is apparent that there exists a need in the art for a method of treating CNS disorders which involves replacing cells lost to injury or disease, but that does not involve invasive transplantation procedures and the disadvantages attendant with such procedures. In addition, due to the problems associated with pharmaceutical compounds, there exists a need in the art for a method of treating CNS disorders which involves the direct delivery of compounds to the CNS by other CNS cells thereby circumventing the use of infusion pumps and the blood-brain barrier.

Therefore, it is an object of the invention to provide compositions that are used to induce proliferation, differentiation and/or migration of endogenous CNS precursor cells and/or to genetically modify CNS precursor cells *in situ*.

Thus, the present invention provides for the use of a combination comprising epidermal growth factor and fibroblast growth factor in the manufacture of a pharmaceutical composition for treatment of neurological disease, disorder and/or injury.

Another object of this invention is to provide a method for increasing the *in vitro* yield of stem cells by epigenetic manipulation of endogenous precursor cells.

These objects are accomplished by the methods disclosed below. Additional objects and features of the invention will be apparent to those skilled in the art from the following detailed description and appended claims when taken in conjunction with the figures.

Also disclosed is a method of *in vitro* proliferation of precursor cells comprising the steps of administering a combination of fibroblast growth factor and epidermal growth factor to a CNS ventricle of a living mammal, removing tissue lining the CNS ventricle, dissociating the tissue to separate precursor cells, and exposing the precursor cells to a culture medium containing a growth factor that induces proliferation of the cells.

A method of treating a mammal suffering from a neurological disorder caused by lost or damaged neural cells is described wherein one or more growth factors is administered to a CNS ventricle of the mammal to induce the *in situ* proliferation, migration, and differentiation of precursor cells lining the ventricle so as to replace the lost or damaged neural cells.

### Brief Description of the Figures

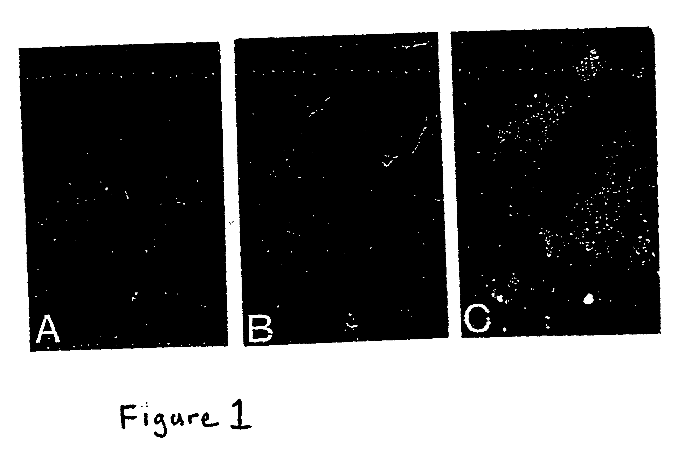

**Figures 1a-1c** show differentiated cells that were generated from stem cells isolated from the adult conus medullaris. Tissue containing the conus medullaris was dissected from adult mice. The stem cells were isolated and cultured in the presence of 20 ng/ml of EGF and bFGF together. They generated neurons, astrocytes, and oligodendrocytes as indicated by triple label indirect immunocytochemistry with antisera to MAP-2 (Fig. 1A), GFAP (Fig. 1B) and O4 (Fig. 1C) respectively.

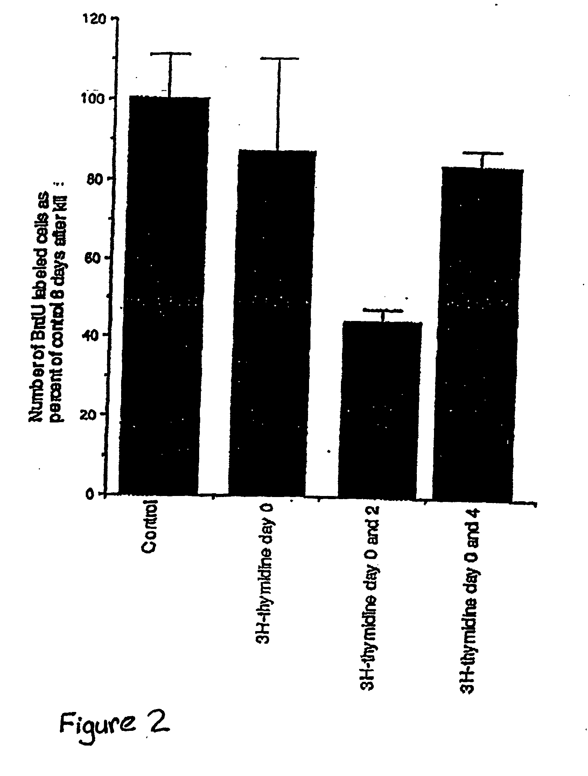

**Figure 2** shows the number of cells proliferating in the subependyma after various thymidine kill treatments. Control mice received no tritiated-thymidine (3H-thymidine) and were considered to have 100% of a proliferating subependymal cell population. Animals that received a series of injections on day 0 in order to kill the constitutively proliferating population of cells had 85% of control values on day 8. Animals that received a series of 3H-thymidine injections on day 0 and on day 2 had 45% of control values 8 days after the last injection (day 10). Animals that received a series of 3H-thymidine injections on day 0 and day 4 had 85% of control values on day 12. This suggests that stem cells are recruited into the proliferative mode two days after the initial kill and that most have become quiescent again by day 4.

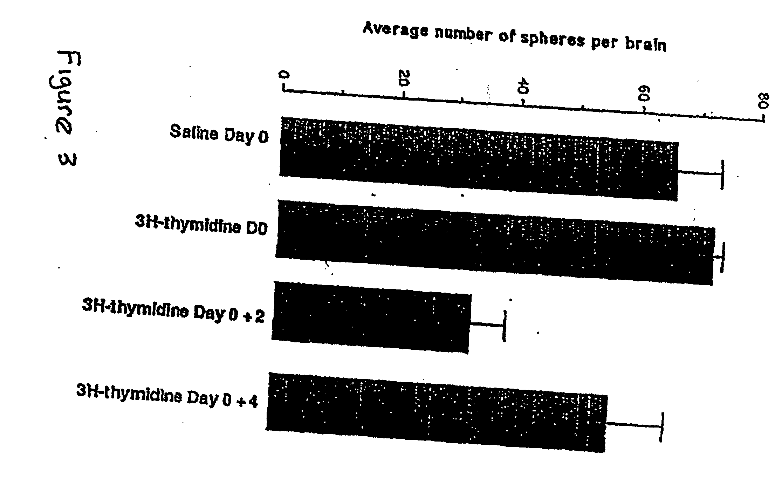

**Figure 3** shows the number of neurosphere formed *in vitro* from animals subjected to various 3H-thymidine treatments *in vivo*. Animals received the same series of injections as illustrated in Figure 2. Control animals received saline injections. Mice were sacrificed 16-20 hours following the last 3H-thymidine injection and the brains were removed and cultured in order to assess the number of neurospheres that formed following each thymidine kill treatment. Animals that received 3H-thymidine on day 0 (which kills the normally proliferating subependymal cells) formed the same number of spheres as control animals, suggesting that the constitutively proliferating subependymal cells are not the source of stem cells isolated *in vitro.* Animals that received a second series of injections on day 2 formed 45% the number of neurospheres relative to control values. Animals that received a second series of injections on day 4 formed nearly as many neurospheres as the control animals, suggesting that the neurosphere-forming multipotent stem cell, which is isolated *in vitro* in the presence of growth factors such as epidermal growth factor, comes from the relatively quiescent stem cell population within the subependyma *in vivo.*

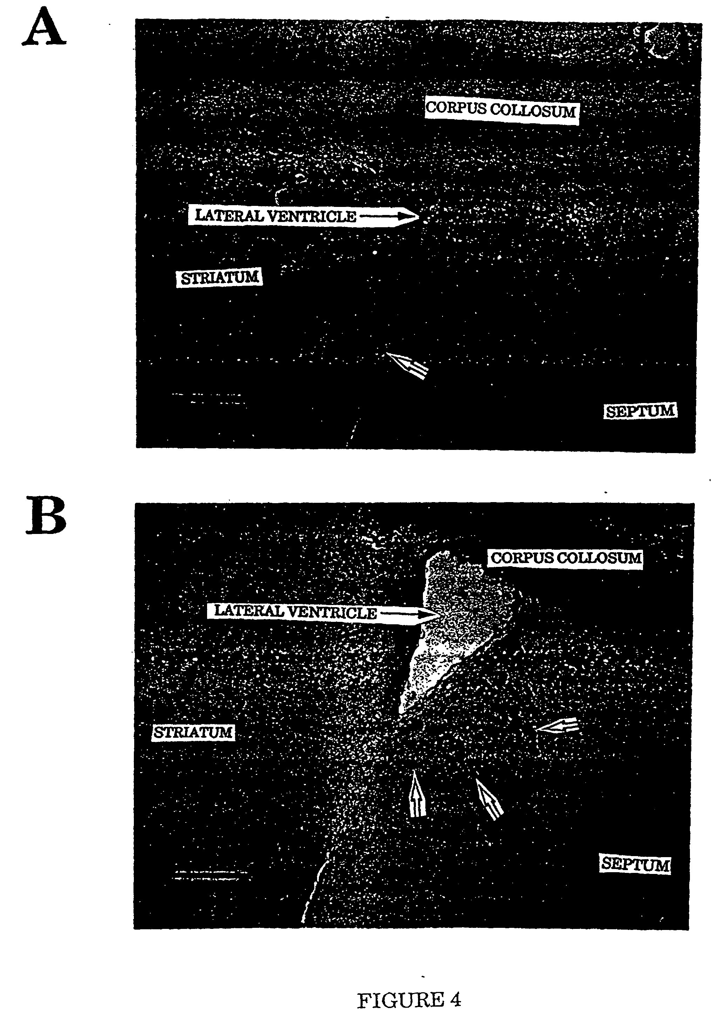

**Figures 4a and 4b** demonstrate the proliferation and migration of retrovirally-labelled precursor cells in the subependyma in response to EGF-infusion *in vivo* into the lateral ventricle. The animals of Figure 4a and 4b had replication incompetent retroviruses containing the β-galactosidase gene injected into the lateral ventricles of their forebrains. Additionally, the animal of Figure 4b had an osmotic mini-pump filled with 33 µg/ml EGF implanted at the same site as the injection. Six days later the brains of both animals were fixed and processed for β-gal histochemistry. With the control animal (Fig. 4a) retrovirally labelled cells (open arrow) resided closely to the lateral ventricle wall. The animal that received EGF infusion had many more labelled cells. Additionally the proliferated cells of the EGF-infused animal had migrated away from the lateral ventricle wall (bar represents 200 µm).

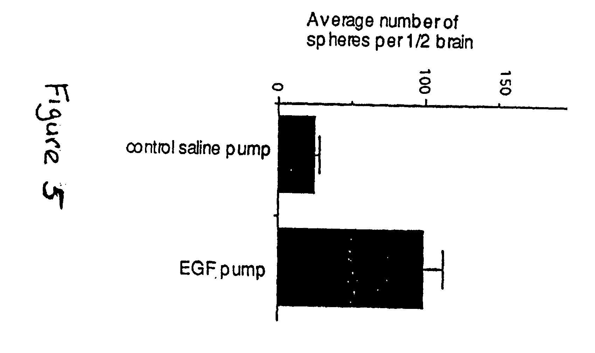

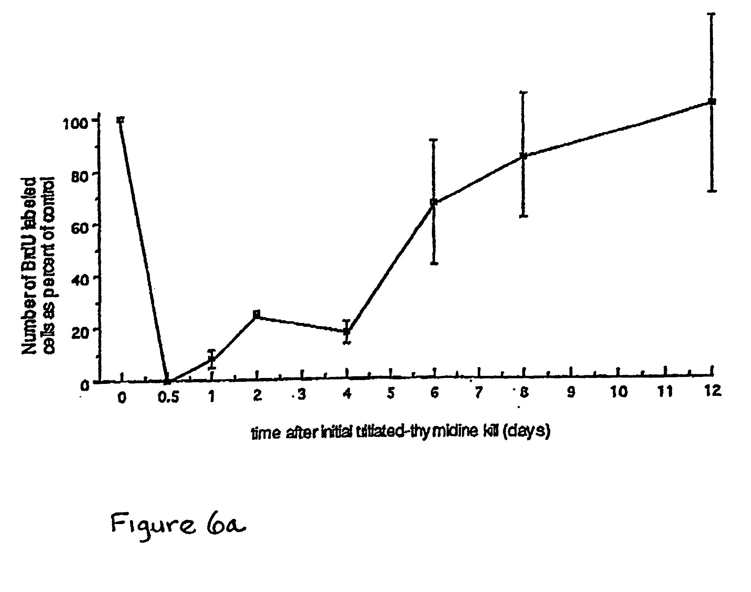

**Figure 5** demonstrates that EGF infusion *in vivo* into the lateral ventricle dramatically increases the number of neurospheres formed *in vitro.* Animals were treated with 33 µg/ml EGF-filled infusion pumps for 6 days prior to sacrifice. Subsequent culturing of the lateral ventricle walls in the presence of 20 ng/ml EGF resulted in a 4-fold increase in the number of neurospheres formed after 9 days *in vitro* as compared to control animals that received infusions of saline for the same 6 days.

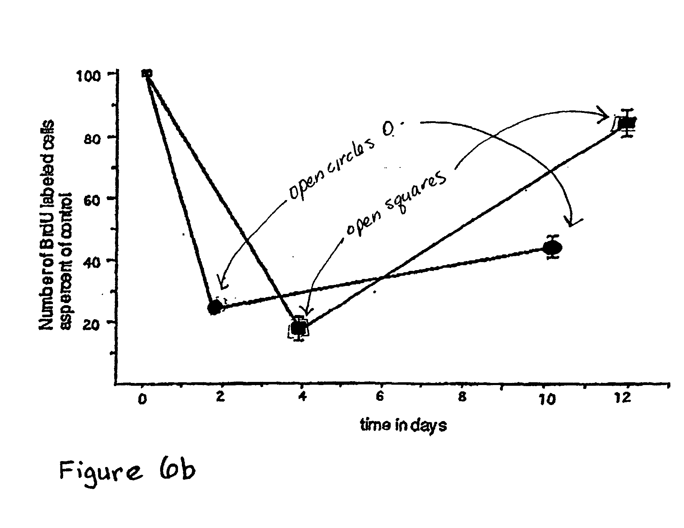

**Figures 6a and 6b** depict the time it takes to reconstitute the number of proliferating cells in the subependyma after a single series of 3H-thymidine injections (Fig. 6a) and after a second series of 3H-thymidine injections given 2 days after the first series (Fig. 6b). As shown in Figure 6a, one day after a single series of injections only 10% of the subependymal cells were proliferating relative to control values (no 3H-thymidine injection). There was a continual increase in the number of proliferating cells over time and by 8 days, the number of proliferating subependymal cells had returned to control values. When a second series of injections was given two days after the first series, as shown in Figure 6b (open circles) the number of stem cells was depleted and the proliferating population was reduced to 50% of control values. When the second series of injections was given four days after the first series (open squares) the number of stem cells which were recruited to repopulate the subependyma had exited from the cell cycle and were no longer proliferating. Hence, they were not killed by the second series of injections on day 4. In this case, the number of proliferating subependymal cells returned to 85% of the control values 8 days after the second series of injections.

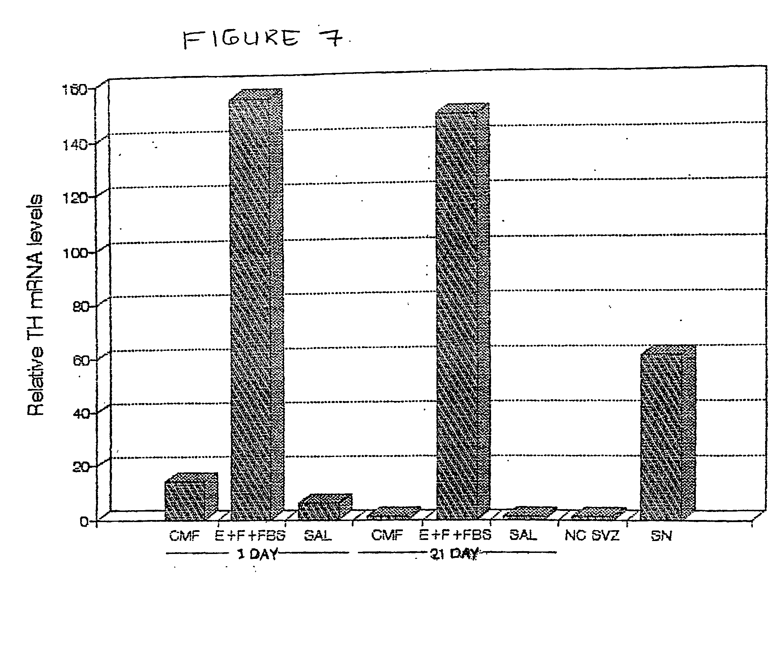

**Figure 7** shows the relative amounts of tyrosine hydroxylase (TH) mRNA in treated and untreated subventricular zones (SVZs) and in the substantia nigral region (SN) of CD₁ mice, one day and twenty one days after administration of growth factors. CMF = conditioned medium from the rat B49 glial cell line + FGF (25 µg/ml) + heparan sulfate (10 IU/ml). E+F+FBS = EGF (25 µg/ml) + bFGF (25 µg/ml) + heparan sulfate (10 UI/ml) + 25% fetal bovine serum. SAL = saline. NC SVZ = non-cannulated subventricular zone.

### Detailed Description of the Invention

### Definitions

The term "stem cell" refers to a relatively quiescent undifferentiated cell which is capable of proliferation and giving rise to more stem cells having the ability to generate a large number of progenitor cells that can in turn give rise to differentiated, or differentiable daughter cells.

The term "neural stem cell" refers to multipotent stem cells capable of producing progeny that are capable of differentiating into neurons, astrocytes and oligodendrocytes.

The term "progenitor cell" refers to an undifferentiated cell derived from a CNS stem cell. The progenitor cell has limited proliferative ability and cannot self-renew. It is committed to a particular path of differentiation and will, under appropriate conditions, eventually differentiate into neurons, astrocytes or oligodendrocytes.

The term "oligodendrocyte" refers to a differentiated glial cell which forms the myelin surrounding axons in the central nervous system (CNS). Oligodendrocytes are of the phenotype galactocerebroside (+), myelin basic protein (+), and glial fibrillary acidic protein (-) [GalC(+), MBP(+), GFAP(-)].

The term "neuron" refers to a differentiated neuronal cell having the phenotype neuron specific enolase (+), neurofilament (+), microtubule associated protein (+), or Tau-1 (+) [NSE(+), NF (+), MAP-2 (+), or Tau-1 (+)].

The term "astrocyte" refers to a differentiated glial cell that is GFAP(+), GalC(-), and MBP(-).

The term "neurosphere" refers to a cluster of cells derived from neural stem cells and cultured *in vitro.* At least some of the cells are of the nestin (+) phenotype. The cluster is comprised of stem cells and/or progenitor cells and may or may not include differentiated cells.

The term "precursor cells" refers to the living cells modified or manipulated by the methods of the instant invention that are derived from neural stem cells, *in vivo* or *in vitro,* and includes both progenitor and stem cells. *In vitro,* precursor cells typically grow in the form of neurospheres, but may exhibit different growth patterns depending upon culture conditions. Precursor cells are found in the subependyma but are not limited to this CNS region.

The term "growth factor", refers to a biological factor such as a protein, peptide or other molecule having a growth, proliferative, differentiative, trophic, or regulatory effect (either singly or in combination with other growth factors) on precursor cells.

Examples of growth factors include acidic and basic fibroblast growth factors (aFGF, bFGF), activin, amphiregulin. the Bcl-2 gene product, bone morphogenic protein (BMP-2), brain-derived neurotrophic factor (BDNF), ciliary neurotrophic factor (CNTF), epidermal growth factor (EGF), an EGF-like ligand, glial-derived neurotrophic factor (GDNF), heparin-like molecules such as heparan sulphate, insulin-like growth factor (IGF₋₁), interleukins, macrophage inflammatory proteins (MIP-1α, MIP-1β and MIP-2), nerve growth factor (NGF), platelet-derived growth factor (PDGF), retinoic acid, thyrotropin releasing hormone (TRH), transforming growth factors alpha and beta (TGFα, TGFβ), and tumor necrosis factor alpha (TNFα), and the like.

The term "neurological agent" refers to any biologically active substance useful for the functioning of CNS cells. For example, the term encompasses neurotransmitters, neurotransmitter receptors, growth factors, growth factor receptors, and the like, as well as enzymes used in the synthesis of these agents.

The term "ventricle" refers to any cavity or passageway within the central nervous system through which cerebral spinal fluid flows. Thus, the term not only encompasses the lateral, third, and fourth ventricles, but also encompasses the central canal and the cerebral aqueduct.

The term "ventricular tissue" refers to the tissue lining CNS ventricles and includes the subependyma which comprises a collection of undifferentiated cells including CNS stem cells and progenitor cells.

The term "constitutively proliferating population" refers to the population of dividing cells located within the subependyma of the lateral ventricles of the adult mammalian forebrain [as outlined by Smart; *J. Comp. Neurol. 116*:325, (1961)], and makes up approximately 33% of the cells in some regions of the subependyma [Morshead and van der Kooy, *J. Neurosci. 12*:249, (1992)].

The term "genetic material" refers to the RNA and DNA that encodes a neurological agent selected from the group consisting of growth factors, growth factor receptors, neurotransmitters, neurotransmitter receptors, neuropeptides, growth factor synthesizing enzymes, and neurotransmitter synthesizing enzymes. The genetic material is used to infect precursor cells *in vitro* or *in vivo* using genetic modification techniques known in the art.

### Description of the Preferred Embodiments

### In Vitro Isolation and Characterization of Stem Cells From the Adult Mammalian CNS

CNS stem cells have been reported and their potential use described [Reynolds and Weiss, *Science* 255:1707 (1992); Reynolds *et al*., *J. Neurosci 12*:4565 (1992); Reynolds and Weiss, *Restorative Neurology and Neuroscience 4*:208 (1992); Reynolds and Weiss, *Neuronal Cell Death and Repair*, ed. Cuello (1993)]. Additionally, the utility of these cells is described in published PCT applications no. WO 93/01275, WO 94/16718, WO 94/10292, and WO 94/09119. Like stem cells found in other mammalian tissues, the CNS stem cell is capable of self maintenance and generating a large number of progeny -- these include neurons, astrocytes and oligodendrocytes.

CNS precursor cells can be isolated and cultured from the adult mammalian CNS by the methods described in Example 2 below and in the Reynolds and Weiss publications and PCT applications referenced above. in brief, the epidermal growth factor (EGF)-responsive stem cell, is grown in a culture medium in the presence of EGF with FGF or bFGF. The medium is preferably a defined serum-free medium. Further growth factors which may be used for inducing proliferation include any trophic factor which allows precursor cells to proliferate, including any molecule which binds to a receptor on the surface of the cell to exert a trophic, or growth-inducing effect on the cell. Growth factors having a proliferative effect include, EGF, amphiregulin, aFGF, bFGF, and TGFα. The combination of the invention is EGF with aFGF or bFGF.

Certain growth factors have a regulatory effect on the proliferation of precursor cells. These growth factors can be used in combination with the above-mentioned proliferation-inducing combination of growth factors to regulate proliferation, for example by increasing or slowing down proliferation or by altering the phenotypes of the precursor cell progeny. Growth factors that have a regulatory effect on proliferation include heparan sulphate, transforming growth factor beta (TGFβ), activin, bone morphogenic protein (BMP-2), ciliary neurotrophic factor (CNTF), retinoic acid, tumor necrosis factor alpha (TNFα), macrophage inflammatory proteins (MIP-1α, MIP-1β and MIP-2), nerve growth factor (NGF), platelet derived growth factor (PDGF), interleukins, and the Bcl-2 gene product.

The proliferation inducing combination of growth factors induces the proliferation of a CNS stem cell which divides to give rise to a cluster of undifferentiated precursor cells. The clusters of cells are not immunoreactive for GFAP, neurofilament (NF), neuron-specific enolase (NSE) or MBP. However, precursor cells within the cluster are immunoreactive for nestin, an intermediate filament protein found in undifferentiated CNS cells. The nestin marker was characterized by Lehndahl *et al.* [*Cell 60*:585-595 (1990)]. The mature phenotypes associated with the four cell types which may be differentiated from the progeny of the precursor cells are predominantly negative for the nestin phenotype.

In the continued presence of a mitogen such as EGF or the like, precursor cells within the neurosphere continue to divide resulting in an increase in the size of the neurosphere and the number of undifferentiated cells [nestin(+), GFAP(-), NF(-), NSE (-), MBP (-)]. At this stage, the cells are non-adherent and tend to form the free-floating clusters characteristic of neurospheres. However, culture conditions may be varied so that while the precursor cells still express the nestin phenotype, they do not form the characteristic neurospheres. After removal of the mitogen, the cells adhere to the substrate (poly-ornithine-treated plastic or glass), flatten, and begin to differentiate into neurons and glial cells. At this stage the culture medium may contain serum such as 0.5-1.0% fetal bovine serum (FBS). Within 2-3 days, most or all of the precursor cells begin to lose immunoreactivity for nestin and begin to express antigens specific for neurons, astrocytes or oligodendrocytes as indicated by immunoreactivity for MAP-2, GFAP and GalC, respectively.

The identification of neurons is also accomplished using immunoreactivity for neuron-specific enolase (NSE), neurofilament, and the neuron specific protein tau-1. Because these markers are highly reliable, they will continue to be useful for the primary identification of neurons, however neurons can also be identified based on their specific neurotransmitter phenotype.

Using dual-label immunofluorescence and immunoperoxidase methods, differentiated neurosphere cultures can be analyzed for the expression of neurotransmitters, or in some cases for the enzymes responsible for the neurotransmitter synthesis. Alternatively, *in situ* hybridization histochemistry can be performed using cDNA or RNA probes specific for the peptide neurotransmitter or the neurotransmitter synthesizing enzyme mRNAs. These techniques can be combined with immunocytochemical methods to enhance the identification of specific phenotypes. If necessary, the antibodies and molecular probes discussed above can be applied to western and northern blot procedures respectively to aid in the cell identification.

Cells that do not express intermediate filaments specific for neurons or for astrocytes, begin to express markers specific for oligodendrocytes in a correct temporal fashion. That is, the cells first become immunoreactive for O4 (a cell surface antigen), galactocerebroside (GalC, a myelin glycolipid) and finally, myelin basic protein (MBP). These cells also possess a characteristic oligodendrocyte morphology.

CNS stem cells have been isolated from a variety of adult CNS regions, including the conus medullaris (see Fig. 1a-1c), cervical and thoracic spinal cord, brain stem, and hypothalamus. In each of these cases the isolated CNS stem cell exhibits self-maintenance and generates a large number of progeny which include neurons, astrocytes and oligodendrocytes. Thus stem cells are present in multiple regions of the adult mammalian CNS.

### Characterization and Manipulation of Stem Cells from the Adult Mammalian CNS In Vivo

The techniques that are described above to isolate, proliferate, and differentiate, CNS stem cells *in vitro* can be adapted to *in* vivo techniques, as described below, to achieve similar results. Such *in vivo* manipulation and modification of these cells allows cells lost, due to injury or disease, to be endogenously replaced, thus obviating the need for transplanting foreign cells into a patient. Additionally, the cells can be modified or genetically engineered *in vivo* so that they express various biological agents useful in the treatment of neurological disorders.

Normally, the adult mammalian CNS is mitotically quiescent *in vivo* with the exception of the subependymal region lining the lateral ventricles in the forebrain. This region contains a subpopulation of constitutively proliferating cells with a cell cycle time of 12.7 hours. BrdU and retroviral labelling of the proliferating cells reveal that none of the newly generated cells differentiate into mature neurons or glia nor do they migrate into other CNS regions (Morshead and Van der Kooy, *supra*).

The continual proliferation and maintenance of a constant number of cells within the subependyma is explained by two mechanisms. The death of one of the daughter cells after each division maintains the proliferating population at a constant number. The constitutively dividing population eventually dies out and a subpopulation of relatively quiescent cells within the subependyma is able to repopulate the constitutively dividing population. This stem cell-like mode of maintaining the proliferative subependymal population is analogous to other tissues where cells have a short life span and are repopulated by a subpopulation of relatively quiescent cells referred to as stem cells.

As detailed in Example 1, experiments utilizing retrovirus infection of these proliferating cells *in vivo* and subsequent β-galactosidase (β-gal) reporter gene expression as a non-diluting marker show that with increasing adult mice survival times (of up to 28 days post retrovirus infection) there is a progressive loss of β-gal positive subependymal cells. Relative to 1 day survival animals, 6 days following retrovirus injection there is a 45% loss of β-gal positive cells and 28 days following retrovirus infection there is a 97% loss. Using nested polymerase chain reaction (PCR) to identify single cells containing retroviral DNA it was determined that the loss of β-gal expressing cells is due to the loss of the retrovirally infected cells through cell death, not due to the turn-off of β-gal expression.

Intraperitoneal injections of BrdU (a thymidine analog that is incorporated into the DNA of dividing cells) reveal that 33% of the cells within some regions of the subependyma make up the normally dividing population (see Example 9 below). The number of BrdU labelled cells decreases over time. By 30 days after BrdU labelling, only 3% of the dividing cells are still labelled. The heavy labelling of a small number of cells 30 days after BrdU injections demonstrates that the labelled cells were dividing at the time of the injections and suggests that they were relatively quiescent for the 30 day period. This indicates that these cells are stem cells rather than cells of the constitutively proliferating population.

The above two examples support the hypothesis that the maintenance of the constant number of proliferating subependymal cells seen throughout adult life requires the presence of a relatively quiescent stem cell that proliferates sporadically to replenish the constitutively proliferating population and to self-renew.

As detailed in Example 5, the constitutively dividing subependymal cells can be killed off by injecting high doses of radioactive thymidine for the duration of the cell cycle at intervals less than S-phase duration. At one day post-kill the proliferating population is 10% of controls and by 8 days the proliferating population is back to control levels. If the replenished population is due to the recruitment of normally quiescent cells into the proliferative mode, then a second kill at the time that stem cells are generating progeny to repopulate the subependyma should alter the number of cells within the constitutively proliferating population. When a second kill is done 2 days after the initial kill, 8 days later the constitutively proliferating population is only 45% of the control values (animals receiving no thymidine kill treatment) or animals that received only one kill at day 0 (the time of the first kill) (see Fig. 6b). The reduction in the number of proliferative cells in the subependyma is maintained at 63% even at 31 days after the second kill. When a second kill is done on day 4, the proliferating population returns to 85% of control values 8 days later. These results suggest that the normally quiescent stem cell is recruited into the proliferative mode within the first two days after the initial kill and that by 4 days the stem cell no longer needs to be recruited to repopulate the subependyma (see Fig. 2).

As detailed in Example 6 below, an experiment was performed to determine whether the *in vitro* stem cell is derived from the constitutively proliferating population or from the quiescent population. Animals were treated in one of the following ways:

| | |
|---|---|
| Group 1. | Control |
| High doses of radioactive thymidine were given on: | |
| Group 2. | day 0 |
| Group 3. | day 0 and day 2 |
| Group 4. | day 0 and day 4 |

Following the last injection animals were killed and stem cells isolated from the striatum (including the subependymal region) via the methods described in Example 2 below.

In groups 2-4 the constitutively proliferating population was killed. In group 3 stem cells that are recruited into the cell cycle to repopulate the subependymal proliferating cells were also killed.

| Number of Neurospheres produced *in vitro:* | |
|---|---|
| Group 1. | 100% (Control) |
| Group 2. | 100% |
| Group 3. | 45% |
| Group 4. | 85% |

These results demonstrate that when you eliminate nearly all of the constitutively proliferating cells in the subependyma this does not affect the number of stem cells that can be isolated and proliferated *in vitro* (group 1 vs. group 2 and 4). However, when the normally quiescent cells are killed when they are recruited to repopulate the subependyma (as with group 3) the number of stem cells that can be isolated *in* *vitro* is significantly reduced (group 3 vs. group 1 and 2). By 4 days after the first kill most of the stem cells themselves are no longer turning over and as a result are not killed by the second series of tritiated thymidine injections (hence, only a 15% reduction [group 4] compared to 55% reduction [group 3]).

The above results demonstrate that *in vitro* stem cells are derived from the quiescent population of subependymal cells *in vivo* (see Fig. 3). This also explains why stem cells can be derived from CNS ventricular regions, other than the forebrain, which do not have the subpopulation of constitutively proliferating cells. Thus, having determined the relationship between the constitutively proliferating population of subependymal cells and the relatively quiescent stem cells of the lateral ventricles, one can take advantage of the characteristics of these cell populations to more effectively manipulate them *in vivo.* Such techniques allow the use and manipulation of endogenous cells for the treatment of a variety of neurological disorders. Some factors that influence proliferation and differentiation of the cells *in vitro* also achieve the same effects *in vivo*. Thus, for example, cells can be manipulated *in situ* by the administration of growth factors and combinations of growth factors that result in specified outcomes as determined by *in vitro* studies. For example, the *in vitro* differentiation methods disclosed in WO 94/10292 in which cells can be induced to follow a particular differentiative pathway, may be modified using the methods described herein to achieve similar results *in vivo.* The growth factors can be administered to a ventricle by stereotactic injection, implantation of an osmotic pump, or any other suitable method.

The quiescent neural stem cells are located throughout the CNS near ventricular regions. Within the forebrain are the lateral (first and second) ventricles. The third ventricle is a cavity of the lower part of the forebrain. The cavity of the midbrain is the tubular shaped cerebral aqueduct and connects the third ventricle with the cavity within the hindbrain, the fourth ventricle. The central canal is the passageway for cerebral spinal fluid within the spinal cord.

The fact that CNS stem cells are located in the tissues lining ventricles offers several advantages for the modification and manipulation of these cells *in situ* and the ultimate treatment of various neurological diseases, disorders, and injury that affect different regions of the CNS. Therapy for these can be tailored accordingly so that stem cells surrounding ventricles near the affected region would be manipulated or modified *in situ* using the methods described herein. The ventricular system is found in nearly all brain regions and thus allows easier access to the affected areas. If one wants to modify the stem cells by exposing them to a composition comprising a growth factor or a viral vector, it is relatively easy to implant a device that administers the composition to the ventricle. For example, a cannula attached to an osmotic pump may be used to deliver the composition. Alternatively, the composition may be injected directly into the ventricles. This would allow the migration of the CNS stem cell progeny into regions which have been damaged as a result of injury or disease. Furthermore, the close proximity of the ventricles to many brain regions would allow for the diffusion of a secreted neurological agent by the stem cells or their progeny.

For treatment of Huntington's Disease, Alzheimer's Disease, Parkinson's Disease, and other neurological disorders affecting primarily the forebrain, growth factors or other neurological agents would be delivered to the ventricles of the forebrain to affect *in situ* modification or manipulation of the stem cells. For example, Parkinson's disease is the result of low levels of dopamine in the brain, particularly the striatum. It would be advantageous to induce a patient's own quiescent stem cells to begin to divide *in vivo* and to induce the progeny of these cells to differentiate into dopaminergic cells in the affected region of the striatum, thus locally raising the levels of dopamine. Normally the cell bodies of dopaminergic neurons are located in the substantia nigra and adjacent regions of the mesencephalon, with the axons projecting to the striatum. Prior art pertaining to the induction of dopaminergic neurons in the brain focusses on manipulations of the mesencephalic tissue. Prior art methods for treating Parkinson's disease usually involves the use of the drug L-Dopa, to raise dopamine levels in the striatum. However, there are disadvantages with this treatment including drug tolerance and side effects. Also, embryonic tissues that produce dopamine have been transplanted into the striatum of human Parkinsonian patients with reasonable success. However, in addition to being a controversial method of treatment, there are risks associated with implantation of foreign tissue including tissue rejection and infection.

The methods and compositions of the present invention provide an alternative to the use of drugs and the controversial use of embryonic tissue for treatment of Parkinson's disease. The level of dopamine cells in the striatum is increased by the administration of a composition comprising growth factors to the lateral ventricle. A particularly preferred composition comprises a combination of EGF, bFGF, and heparan sulphate. The composition preferably also comprises serum. After administration of this composition, there is a significant increase in the transcription of messenger RNA (mRNA) for tyrosine hydroxylase (TH) (a marker for dopamine cells) in the subventricular region of the striatum, an area which normally does not contain dopaminergic cell bodies. These methods and results are described in detail in Example 11. As detailed in Example 12, dual labelling tissue to show the distribution of BrdU+ and TH+ cells indicates that, in response to the *in vivo* administration of growth factors, TH+ cell bodies occur in striatal tissue. Many of these newly generated TH+ cells are also BrdU+.

For the treatment of multiple sclerosis and other demyelinating or hypomyelinating disorders, and for the treatment of Amyotrophic Lateral Sclerosis or other motor neuron diseases, growth factors or other neurological agents would be delivered to the central canal. Other diseases and disorders which would be treated by the methods of the present invention are disclosed in PCT application WO 93/01275.

In addition to treating CNS tissue immediately surrounding a ventricle, a viral vector, DNA, growth factor, or other neurological agent can be easily administered to the lumbar cistern for circulation throughout the CNS.

Under normal conditions subependymal precursors do not differentiate or migrate, rather, their fate appears to be cell death after an undefined number of cell divisions (Morshead and Van der Kooy, *supra*). This explanation is also supported by PCR evidence, as described above. Injection of growth factors into the lateral ventricle alters this fate. As described in more detail in Example 1 below, retroviruses were injected into the lateral ventricles for six consecutive days. Implanting cannulae attached to EGF-filled osmotic pumps into the lateral ventricles on the same day as (and 1 or 6 days following) retrovirus injection results in an increase in the total number of RV-β-gal labelled cells 6 days later (from an average of 20 cells/brain to 150 cells/brain).

It is known from the PCR experiments described above that 6 days following retroviral injection no cells exist that contain non-expressed retroviral DNA. Thus these results indicate that the EGF-induced increase in β-gal positive cell number is due to the expansion of the clone size of the retrovirally labelled constitutively proliferative population. It is also possible that part of this increase is due to the activation by EGF of a relatively quiescent stem cell.

Interestingly, this expansion of the number of β-gal labelled cells is accompanied by the migration of these cells away from the subependymal medially and laterally (and possibly rostrally and caudally) up to a distance of approximately 400 µm (see Fig. 4), with subsequent differentiation. Thus, infusion of EGF or similar growth factors induces the proliferation, migration and differentiation of neural stem cells and progenitor cells *in vivo*, and can be used therapeutically to replace neural cells lost due to injury or disease. In the invention EGF and FGF are administered together or sequentially.

The normal fate of the constitutively proliferating cell population (i.e. cell death) can be altered by administering Bcl-2 or genetically modifying the cells with the bcl-2 gene. The gene product is known to prevent programmed cell death (apoptosis) in a variety of cell types. Similar to the EGF experiments, a clonal expansion of the constitutively proliferating cell population is achieved.

The *in vivo* techniques described herein can be used to genetically modify CNS stem cells and progenitor cells. Any useful genetic modification of the cells is within the scope of the present invention. For example, the cells may be modified to produce or increase production of a neurological agent such as a neurotransmitter, growth factor, or the like. The genetic modification is performed either by infection of the cells lining CNS ventricular regions with recombinant retroviruses or transfection using methods known in the art [see Maniatis *et al., Molecular Cloning: A Laboratory Manual,* Cold Spring Harbor Laboratory, N.Y. (1982)]. Any method of genetic modification, now known or later developed can be used. With direct DNA transfection, cells could be modified by particle bombardment, receptor mediated delivery, and cationic liposomes. When chimeric gene constructs are used, they generally will contain viral, for example retroviral long terminal repeat (LTR), simian virus 40 (SV40), cytomegalovirus (CMV); or mammalian cell-specific promoters such as tyrosine hydroxylase (TH), dopamine β-hydroxylase (DBH), phenylethanolamine N-methyltransferase (PNMT), choline acetyltransferase (ChAT), glial fibrillary acidic protein (GFAP), neuron-specific enolase (NSE), the neurofilament (NF) proteins (NF-L, NF-M, NF-H, and the like) that direct the expression of the structural genes encoding the desired protein.

If a retroviral construct is to be used to genetically modify normally quiescent stem cells, then it is preferable to induce the proliferation of these cells using the methods described herein. For example, an osmotic infusion pump could be used to deliver growth factors to the central canal several days prior to infection with the retrovirus. This assures that there will be actively dividing neural stem cells which are susceptible to infection with the retrovirus.

When the genetic modification is for the production of a biologically active substance, the substance will generally be one that is useful for the treatment of a given CNS disorder. For example, it may be desired to genetically modify cells so they secrete a certain growth factor product. Growth factor products useful in the treatment of CNS disorders include, but are not limited to, nerve growth factor (NGF), brain-derived neurotrophic factor (BDNF), the neurotrophins (NT-3, NT-4/NT-5), ciliary neurotrophic factor (CNTF) and other cytokines, amphiregulin, bFGF, aFGF, EGF, TGFα, TGFβ, PDGF, IGFs, and the interleukins.

Cells can also be modified *in vivo* to express a certain growth factor receptor (r) including, but not limited to, p75 low affinity NGFr, CNTFr, the *trk* family of neurotrophin receptors (*trk*A, *trk*B, *trk*C), EGFr, FGFr, and amphiregulin receptors.

Cells can be engineered to produce various neurotransmitters or their receptors such as serotonin, L-dopa, dopamine, norepinephrine, epinephrine, tachykinin, substance-P, endorphin, enkephalin, histamine, N-methyl D-aspartate (NMDA), glycine, glutamate, γ-amino butyric acid (GABA), acetylcholine, and the like. Useful neurotransmitter-synthesizing genes include TH, dopa decarboxylase (DDC), DBH, PNMT, glutamic acid decarboxylase (GAD), tryptophan hydroxylase, ChAT, and histidine decarboxylase. Genes that encode for various neuropeptides, which may prove useful in the treatment of CNS disorders, include substance-P, neuropeptide-Y (NP-Y), enkephalin, vasopressin, vasoactive intestinal peptide (VIP), glucagon, bombesin, cholecystokinin (CCK), somatostatin, calcitonin gene-related peptide (CGRP), and the like.

### Improvement to In Vitro Techniques for Proliferation of CNS Stem Cells

The absolute yield of the number of precursor cells isolated *in vitro* from the mammalian CNS can be increased dramatically by injecting a combination of growth factors: EGF and FGF together, into the ventricles *in situ* as described above. As detailed in Example 10 below, 6 days after infusion of EGF into the lateral ventricle of a mouse forebrain, the walls of the ventricle were removed and the stem cells harvested. Infusion of EGF into the lateral ventricle increased the efficiency of the yield of stem cells that proliferated to form neurospheres.

This technical improvement of *in vitro* technology for the proliferation of CNS stem cells should prove invaluable when stem cells are to be harvested for later transplantation back into a patient, thereby making the initial surgery 1) less traumatic because less tissue would have to be removed and 2) more efficient because a greater yield of stem cells per surgery would proliferate *in vitro.* Additionally, the patient's stem cells, once they have proliferated *in vitro*, could also be genetically modified *in* vitro using the techniques described in PCT application WO 94/16718. The *in vitro* genetic modification may be more desirable in certain circumstances than *in situ* techniques when more control over the infection with the genetic material is required.

In order that the invention described herein may be more fully understood, the following examples are set forth. It should be understood that these examples are for illustrative purposes only and are not to be construed as limiting the scope of the invention in any manner.

### EXAMPLES

### Example 1

A replication incompetent retrovirus containing the β-alactosidase gene [as described in Walsh and Cepko, *Science 241*:1342, (1988)] was injected into the forebrain lateral ventricles of CD1 adult male mice (25-30 g from Charles River). The injected retrovirus was harvested from the BAG cell line (ATCC CRL-9560) according to the method of Walsh and Cepko (*supra*). Mice were anesthetized using 65 mg/kg, i.p. sodium pentobarbital. Unilateral stereotactic injections of 0.2-1.0 µl of retrovirus were injected into the lateral ventricle using a 1 µl Hamilton syringe. The coordinates for injection were AP +4.2 mm anterior to lambda, L ± 0.7 mm, and DV -2.3 mm below dura, with the mouth bar at -2mm below the interaural line.

On the same day as, one day, or six days following the retrovirus injection, an infusion cannulae attached to a 0.5 µl/hour ALZET osmotic mini-pumps filled with 3.3 - 330 µg/ml of EGF were surgically implanted into the lateral ventricles at the identical stereotactic coordinates as stated above. The infusion cannula kits were obtained from ALZA. The infusion cannulae were cut to 2.7 mm below the pedestal. The pumps were secured to the mouse skull by use of acrylic cement and a skull screw contralateral and caudal to the injection site. The osmotic mini-pump was situated subcutaneously under and behind the armpit of the left front paw and connected to the infusion cannula by the means of polyethylene tubing.

Six days following initiation of EGF infusion the animals were sacrificed with an overdose of sodium pentobarbital. Mice were transcardially perfused with 2% buffered paraformaldehyde, and the brains were excised and post fixed overnight with 20% sucrose in 2% buffered paraformaldehyde. Coronal slices were prepared with -20 celsius cryostat sectioning at 30 µm. Slices were developed for β-gal histochemistry as per Morshead and Van der Kooy (*supra*).

Under these conditions, regardless of the day post retrovirus injection, infusion of EGF resulted in an expansion of the population of β-gal labelled cells from an average of 20 cells per brain up to an average of 150 cells per brain and the migration of these cells away from the lining of the lateral ventricles (see Fig. 4). Infusion of basic fibroblast growth factor (bFGF), 33 µg/ml resulted in an increase in the number of β-gal labelled cells, but this increase was not accompanied by any additional migration. Infusion of EGF and FGF together resulted in an even greater expansion of the population of β-gal labelled cells from 20 cells per brain to an average of 350 cells per brain.

These results indicate that FGF may be a survival factor for relatively quiescent stem cells in the subependyma layer, whereas EGF may act as a survival factor for the normally dying progeny of the constitutively proliferating population. The synergistic increase in β-galactosidase cell number when EGF and FGF are infused together further reflects the direct association between the relatively quiescent stem cell and the constitutively proliferating progenitor cell.

### Example 2

The striata, including the subependymal region, of female, pathogen-free CD1 albino mice [3 to 18 month old; Charles River (CF1 and CF2 strains yielded identical results)] were dissected and hand cut with scissors into 1-mm coronal sections and transferred into artificial cerebrospinal fluid (aCSF) containing 124 mM NaCl, 5 mM CKl, 1.3 mM MgCl₂, 2 mM CaCl₂, 26 mM NaHCO₃, and 10 mM D-glucose (pH 7.35, approx. 180 mOsmol), aerated with 95% O₂ - 5% CO₂ at room temperature. After 15 minutes the tissue sections were transferred to a spinner flask (Bellco Glass) with a magnetic stirrer filled with low-Ca²⁺ aCSF containing 124 mM NaCl, 5 mM KCl, 3.2 mM MgCl₂, 0.1 mM CaCl₂, 26 mM NaHCO₃, and 10 mMD-glucose (pH 7.35, approx. 180 mOsmol), aerated with 95% O₂ - 5% CO₂ at 32 to 35° C., containing 1.33 mg/ml of trypsin (9000 BAEE units/mg), 0.67 mg/ml of hyaluronidase (2000 units/mg) and 0.2 mg/ml of kynurenic acid. After 90 minutes, tissue sections were transferred to normal aCSF for 5 minutes prior to trituration. Tissue was transferred to DMEM/F-12 (A:1, Gibco) medium containing 0.7 mg/ml ovomucoid (Sigma) and triturated mechanically with a fire-narrowed pasteur pipet. Cells were plated (1000 viable cells per plate) in noncoated 35 mm culture dishes (Costar) containing serum-free medium [this included a defined hormone mix and salt mixture that included insulin (25 µg/ml), transferrin (100 µg/ml), progesterone (20 nM), putrescine (60µM), and selenium chloride (30 nM); glucose (0.6%); glutamine (2 mM); sodium bicarbonate (3 mM); and HEPES buffer (5 mM)] and EGF [20 ng/ml, purified from mouse sub-maxillary gland (Collaborative Research)] or human recombinant (Gibco/BRL). Cells were allowed to settle for 3-10 minutes after which the medium was aspirated away and fresh DMEM/F-12/hormone mix/EGF was added. After 5-10 days *in vitro* the number of spheres (neurospheres) were counted in each 35 mm dish.

### Example 3.

A retroviral construct containing the human bcl-2 gene and the β-galactosidase gene is microinjected into the adult mouse lateral ventricle. A control mouse is injected with a retroviral construct containing only the β-galactosidase gene. One of the two progeny of each of the constitutively proliferating subependymal cells of the adult lateral ventricle normally dies within a few hours after division. The bcl-2 gene product prevents the programmed death of cells in several other tissues. In the adult subependyma, single cells infected with both the β-galactosidase and bcl-2 genes are marked by expression of both these gene products. These cells are identified in brain tissue slices with antibodies specific to β-galactosidase and human Bcl-2. Proliferating infected subependymal cells so infected produce larger numbers of cells per clone relative to the control. Thus, Bcl-2 induces the survival of the one normally dying progeny of each division of a constitutively proliferating adult subependymal cell. Moreover, the bcl-2 infected progeny migrate out into striatal and septal tissue to produce new neurons and glia. This indicates that EGF and Bcl-2 act as a survival factors for the normally dying progeny of constitutively proliferating adult subependymal cells which generate new neurons and glia *in vivo.*

### Example 4.

A retroviral construct containing the nerve growth factor gene is microinjected (as was the construct in Example 1) to infect the constitutively proliferating adult subependymal cells of the lateral ventricle. Thus, these cells are used to produce an endogenous growth factor in the adult brain. Levels of nerve growth factor produced by the transfected cells are measured directly by radioimmunoassay and (from a direct functional assay) by rescue of basal forebrain cholinergic neurons *in vivo* after axotomy injury in the model developed by Gage and collaborators (P.N.A.S. 83:9231, 1986).

### Example 5.

Adult male CD1 mice received a series of intraperitoneal injections of 3H-thymidine (0.8 ml per injection, specific activity 45-55 mCi/mmole, ICN Biomedical) on day 0 (3 injections, 1 every 4 hours) in order to kill the constitutively proliferating subependymal cells. On day 0.5, 1, 2, 4, 6, 8 or 12, animals received 2 BrdU injections 1 hour apart (see Example 9) and were sacrificed 0.5 hour after the last injection.

It was observed that 10% of the cells were proliferating on day 1 post-kill, and by 8 days the number of proliferating cells had reached 85% (see Fig. 6a), which was not statistically significantly different from control values. Animals were sacrificed and the brains were removed and processed as described in Example 9.

In a second group of animals, 3H-thymidine injections were given on day 0 (3 injections, 1 every 4 hours), followed by an identical series of injections on day 2 or 4. Animals were allowed to survive for 8 days following the second series of injections (days 9, 10 and 12 respectively) at which time they received 2 injections of BrdU and were sacrificed 0.5 hours later. Animals were sacrificed and the brains were removed and processed as described in Example 9.

After the second series of injections on day 2 only 45% of the proliferating population had returned relative to control values. This indicates that the second series of injections given on day 2 had killed the stem cells as they were recruited to the proliferating mode (see Fig. 6b). The second series of injections given on day 4 resulted in a return to control values by day 8 suggesting that by this time, the stem cells were no longer proliferating and hence were not killed by the day 4 series of injections (Fig. 6b).

### Example 6.

Adult male CD1 mice were divided into 4 groups. Group A animals received a series of 3H-thymidine injections on day 0 (3 injections, 1 every 4 hours). Animals in groups B and C received a series of 3H-thymidine injections on day 0 followed by a second series of injections on day 2 or 4. Group D animals received injections of physiological saline instead of 3H-thymidine over the same time course as group A. Animals from all groups were sacrificed by cervical dislocation 16-20 hours following the last series of injections. Brains were removed and the subependyma surrounding the lateral ventricles in the forebrain were dissected and cultured as described in Example 2 in order to assess the number of spheres that formed following each of the injection paradigms. The cells from each brain were plated into two 35 mm wells. At 6 and 8 days *in vitro,* the total number of spheres was counted in each of the 35 mm wells (see Fig. 3).

Control animals that received a series of saline injections formed the same number of spheres as animals that received 3H-thymidine on day 0 (which kills the normally proliferating subependymal cells). This indicates that the constitutively proliferating subependymal cells are not the source of stem cells isolated *in vitro.* Animals that received a second series of injections on day 2 formed 45% the number of spheres (similar to the number of proliferating subependymal cells observed *in vivo).* When a second series of injections was done on day 4, the number of spheres formed in *vitro* was not significantly different from control values, again correlating with the *in vivo* findings. Taken together, this data indicates that the multipotent spheres, which are isolated *in vitro* in the presence of EGF, are formed from the relatively quiescent stem cell population within the subependyma *in vivo.*

### Example 7.

A retroviral construct containing the β-galactosidase gene is microinjected (as in Example 1) into the III ventricle of the diencephalon, IV ventricle of the brain stem and central canal of the spinal cord. Minipumps containing EGF and FGF are then used to continuously administer growth factors for six days (as in Example 1) into the same portion of the ventricular system that the retroviral construct was administered. This produces an increase in the number of β-galactosidase producing cells which survive and migrate out into the tissue near the III ventricle, IV ventricle and central canal of the spinal cord forming new neurons and glia.

### Example 8.

A retroviral construct containing the tyrosine hydroxylase (TH) gene as well as the β-galactosidase gene is microinjected into the adult lateral ventricle as in Example 1. Minipumps containing EGF, FGF, or EGF and FGF together are then used to continuously administer the growth factor(s) into the lateral ventricle for 6 days as in Example 1. As the infected subependymal cells migrate out into the striatum they differentiate into neuronal cells that produce dopamine as measured directly by immunofluorescence with an antibody and (from a direct functional assay) by the ability to overcome the rotational bias produced by unilateral 6-hydroxydopamine lesions.

### Example 9.

Adult male CD1 mice (25-30 g, Charles River) were injected intraperitoneally (i.p.) with bromodeoxyuridine (BrdU, Sigma, 65 mg/kg) every 2 hours for a total of 5 injections in order to label all of the constitutively proliferating cells in the subependyma lining the lateral ventricles in the forebrain. One month later, animals were sacrificed with an overdose of sodium pentobarbital and transcardially perfused using 4% paraformaldehyde. The brains were removed and post-fixed overnight in 4% paraformaldehyde with 20% sucrose. Brain sections were cut on a cryostat (30 um) and collected in a washing buffer [0.1 M phosphate buffered saline (PBS) pH 7.2 with 1% normal horse serum and 0.3% Triton X-100]. Sections were incubated in 1M HCl at 60° C. for 0.5 hours then washed 3 times (10 minutes each) in washing buffer. Following the final wash, sections were incubated in anti-BrdU (Becton Dickinson, 1:25) for 45 hours at 4° C. After incubation in the primary antibody, sections were washed 3 times and incubated for 1 hours in biotinylated horse-anti-mouse secondary antibody (Dimension Lab, 1:50) at room temperature followed by another 3 washes. The sections were then incubated for 1 hour in avidin conjugated FITC (Dimension Lab, 1:50) at room temperature and washed a final 3 times. Sections were mounted on gelatin coated slides, air-dried and coverslipped with Fluoromount. Slides were examined for BrdU positive cells using a NIKON fluorescent microscope. The number of BrdU positive cells was counted within the subependyma surrounding the lateral ventricles in 8 samples in sections between the closing of the corpus callosum rostrally and the crossing of the anterior commissure caudally. It was found that 31 days following the series of BrdU injections, 3% of the subependymal cells were still labeled compared to control animals sacrificed immediately following the series of injections (control 100%).

### Example 10

EGF pumps were implanted as described in Example 1. Animals were sacrificed by cervical dislocation 6 days after the pump was implanted. Brains were removed and stem cells isolated and counted as described in Example 2.

As shown in Figure 5, animals infused with EGF into the lateral ventricles for 6 days prior to sacrifice and brain culturing had 4 times as many spheres forming after 9 days *in vitro* compared to control animals which received saline pumps for the same 6 day period. Thus, infusing EGF into the lateral ventricles *in vivo* prior to removal of precursor cells greatly increases the yield of stem cells which proliferate and form neurospheres *in vitro.*

### Example 11.

Adult male CD₁ mice were anesthetized and placed in a stereotaxic apparatus. A cannula, attached to an ALZET minipump, was implanted into a lateral ventricle of each animal. The minipumps were subcutaneously implanted and were used to deliver (a) conditioned medium (from the rat B49 glial cell line, obtained from D Schubert, Salk Institute) plus bFGF (R&D Systems, 25 µg/ml) plus heparan sulfate (Sigma, 10 IU/ml) (CMF) or (b) EGF (Chiron, 25 µg/ml) plus bFGF (25 µg/ml) plus heparan sulfate (10 IU/ml) plus 25% fetal bovine serum (E+F+FBS) or (c) sterile saline solution (SAL) as a control, into the lateral ventricles. Once batch of animals was sacrificed one day after completion of the delivery regimen and the others were sacrificed twenty days later. The subventricular zones (SVZs) of these mice were dissected out, separating the cannulated, and therefore treated, side from the non-cannulated control sides. The substantia nigra (SN) region of these mice were also recovered. Total RNA was extracted from these tissues using the guanidium thiocyanate acid phenol method [Chomzynski and Sacchi, *Annal. Biochem*. *162:* 156-159, (1987)]. The RNA was then reverse transcribed to produce cDNA. These cDNAs were subject to polymerase chain reaction (PCR) using primers designed to bracket a 254 nucleotide region of the tyrosine hydroxylase (TH) messenger RNA (mRNA) and thermal cycling conditions favoring quantitative amplification. The PCR products were electrophoresed on a 2% agarose gel and then capillary blotted onto a positively charged nylon membrane. Radioactively labelled cDNA probe to TH was hybridized to the filter and detected by autoradiography. The autoradiograph was scanned and analyzed by densitometry to obtain relative levels of mRNA for TH in the SVZs of the cannulated sides in response to the treatments in the non-cannulated control SVZs and in the SN. The results are depicted in the bar graph of Figure 7. In animals analyzed one day after treatment, the administration of E+F+FBS produced an eleven-fold increase in the level of TH mRNA in the SVZ compared to that observed in response to CMF, which in turn was more than twice the level seen with SAL. Twenty one days after treatment, the amount of TH mRNA detected in response to treatment with E+F+FBS was approximately the same as that detected after one day, while CMF and SAL treated SVZs had TH mRNA levels which were below detectable limits and were indistinguishable from the non-cannulated SVZ controls. Under all treatments, the SN had measurable amounts of TH mRNA.

### Example 12

Male CD₁ mice (Charles River, approximately 4 to 6 weeks old) were given intraperitoneal injections of BrdU (Sigma, 120mg/kg) at 2 hour intervals over a 24 hour period, in order to label mitotically active cells. A cannula attached to an ALZET minipump was then implanted unilaterally into a lateral ventricle of each animal in order to deliver compositions a-c (CMF, E+F+FBS, or sterile saline) described in Example 11.

Animals were sacrificed 24 hours after the administration of growth factors using a lethal dose of pentobarbital anesthetic. The animals were then perfused through the heart with 10 ml of ice could 4% paraformaldehyde solution. The brains were removed and tissue in the region extending from the olfactory bulb to the third ventricle, including the striatum, was dissected out and stored overnight at 4°C in a 30% sucrose/4% paraformaldehyde solution. The tissue was then frozen on dry ice and kept at -70°C until processed. 30µm coronal sections were cut using a cryostat and the sections were placed in 12 well porcelain dishes, to which 400 µl of PBS had been added. Sections were rinsed with fresh PBS and incubated overnight with the following primary antibodies: anti-TH (rabbit polyclonal, 1:1000, Eugene Tech International Inc.; or 1:100, Pel-freeze) and mouse anti-BrdU (1:55, Amersham), prepared in PBS/10% normal goat serum/0.3 Triton X-100. Following three rinses in PBS, goat anti-rabbit rhodamine and goat anti-mouse fluorescein (Jackson) were applied in PBS for 50 minutes at room temperature. Sections were then washed three times (10 minutes each) in PBS, placed on glass slides, dried and then coverslipped using Fluorsave (Calbiochem #345789).

The location of dopaminergic neurons was determined by mapping the location of TH-immunoreactive (TH+) cells, or TH+ and BrdU+ cells in relation to the ventricles. In response to saline injections made into the lateral ventricles, the normal population of TH+ fibers were detected in the striatum but no TH+ cell bodies were detected in this region. CMF treatment resulted in the detection of TH+ cell bodies, in addition to the normal population of TH+ fibers, in the striatum and in the region of the third ventricle. E+F+FBS treatment had the most profound effect resulting in the detection of the most TH+ cell bodies. Several of the TH+ cell bodies were also BrdU positive.

### Example 13

The 6-hydroxy-dopamine (6-OHDA) lesion rat model of Parkinson's disease is used to measure the effects of administering various combinations of growth factors to the lateral ventricle. Unilateral 6-OHDA lesions are performed in the rat model and rotation behavior is observed. Minipumps are subcutaneously implanted into the animals as described in Example 11. EGF (Chiron, 25 µg/ml) plus bFGF (25 µg/ml) plus heparan sulfate (10 IU/ml) plus 25% fetal bovine serum is continuously administered to the lateral ventricle. Saline is administered to control animals. The ability to overcome the rotational bias produced by the unilateral 6-OHDA lesions is observed.

### Example 14.

Adult male CD₁ albino mice (30-35g) from Charles River were anaesthetized with sodium pentobarbital (0.40 mL of a 10% solution) and placed in a stereotaxic apparatus. The dorsal aspect of the skull was exposed with a longitudinal incision. Cannulas were inserted into the fourth ventricle (stereotaxic coordinates A/P -7.0, L ± 0.3 D/V -5.8), cerebral aqueduct (A/P -4.8 L ± D/V -2.6), or central canal (D/V -1.5). The cannulae were attached with sterile tubing to subcutaneous positioned ALZET osmotic mini-pumps containing 25 µg/mL EGF (Becton 40001) and/or 25 µg/mL bFGF (R&D Systems 233-FB). Pumps containing sterile saline plus 0.1% mouse albumin (Sigma A3134) were used as controls. The incisions were closed with dental cement.

Six days following surgery mice were injected with 0.15 mL BrdU (Sigma B5002); 18 mg/mL in 0.007% NaOH/0.1M PBS) every 2 hours for 8 hours. They were killed 0.5 hours after the last injection with an anaesthetic overdose, and transcardially perfused with 10 mL of ice-cold sterile saline followed by 10mL of ice-cold Bouin's fixative (5% glacial acetic acid, 9% formaldehyde, 70% picric acid). The cervical spinal cord region was dissected out and post-fixed overnight at 4°C in Bouin's post-fixative solution (9% formaldehyde, 70% picric acid). The following day the tissue was cryoprotected by immersion in 10% sucrose for 2 hours, 20% sucrose for 2 hours, and 30% sucrose overnight. The tissue was frozen in powdered dry ice, mounted in Tissue-Tek (Miles 4583) at -18°C, and 30 µm serial sagittal sections were mounted onto gel-subbed glass slides. Each slide also contained one or more 30 µm coronal sections through the lateral ventricles from the brain of the same animal to serve as a positive control. Slides were kept at - 80°C until processed. ***Immunohistochemistry:*** Slides were rinsed in PBS 3 x 15 minutes in 0.1M PBS at room temperature, hydrolyzed with IN HCl for 60 minutes at 37°C, rinsed for 3 x 15 minutes in 0.1M PBS at room temperature, placed in 6% H₂O₂ in methanol for 30 minutes at room temperature, rinsed for 3 x 15 minutes in 0.1M PBS at room temperature, and incubated in 10% normal horse serum (Sigma H-0146) in 0.1M PBS or 20 minutes at room temperature. Slides were incubated overnight at room temperature in anti-BrdU monoclonal antibody (Becton 7580) that was diluted 1:50 in 0.1M PBS containing 1.5% normal horse serum and 0.3% Triton. The following day the slides were rinsed in PBS for 3 x 10 minutes in 0.1M PBS at room temperature, incubated with biotinylated horse anti-mouse IgG (Vector BA-2000) for 2 hours at room temperature, rinsed for 3 x 15 minutes in 0.1M PBS at room temperature, incubated in ABC reagent (Vector PK-6100) for 2 hours at room temperature, rinsed for 3 x 15 minutes in 0.1M PBS at room temperature, and developed with DAB reagent for 2 to 4 minutes. The slides were coverslipped with Aqua Polymount (Polysciences 18606). The number of BrdU positive cells was counted per cervical spinal cord section. Some BrdU labelled cells were found in the saline control sections. Treatment with either EGF or bFGF resulted in a significant increase in the number of BrdU labelled cells seen compared to control. The combination of EGF plus bFGF produced even a greater amount of BrdU positive cells per section.

### Example 15.

EGF and FGF is infused into the lateral ventricles (see Example 1) (or other CNS ventricular regions that contain quiescent stem cells). The CNS stem cells are then isolated and proliferated *in vitro* using proliferation methods such as that described in Example 2. This procedure results in an increase in the number of stem cells that are generated and an increase in the number of neurospheres (see Fig. 5). Neurospheres generated by this method are used as a source of donor cells for later transplantation into degenerated areas of human adult CNS. Neurospheres can also be proliferated accordingly from a patient's own CNS stem cells and transplanted back into the patient.

## Claims

1. Use of a combination comprising epidermal growth factor and fibroblast growth factor in the manufacture of a pharmaceutical composition for treatment of neurological disease, disorder, and/or injury.

2. The use of claim 1, wherein said pharmaceutical composition additionally comprises other molecules having a growth, differentiative, trophic and/or regulatory effect.

3. The use of claim 2, wherein said molecules are selected from the group consisting of activin, amphiregulin, the Bcl-2 gene product, bone morphogenic protein (BMP-2), brain-derived neurotrophic factor (BDNF), ciliary neurotrophic factor (CNTF), EGF-like ligand, glial-derived neurotrophic factor (GDNF), heparin-like molecules, insulin-like growth factor IGF-1, interleukins, macrophage inflammatory proteins (MIP-1α, MIP-1β and MIP-2), nerve growth factor (NGF), platelet-derived growth factor (PDGF), retinoic acid, thyrotropin releasing hormone (TRH), transforming growth factors alpha and beta (TGFα, TGFβ), and tumor necrosis factor alpha (TNFα).

4. The use of any one of claims 1 to 3, wherein said pharmaceutical composition additionally comprises heparan sulfate.

5. The use of any one of claims 1 to 4, wherein said pharmaceutical composition additionally comprises genetic material for the genetic modification of a neural precursor cell.

6. The use of claim 5, wherein said genetic material is contained within a viral construct.

7. The use of claim 6, wherein said viral construct is a retroviral construct.

8. The use of any one of claims 5 to 7, wherein said genetic material encodes neurological agent selected from the group consisting of growth factors, growth factor receptors, neurotransmitters, neurotransmitter receptors, neuropeptides, growth factor synthesizing enzymes and neurotransmitter synthesizing enzymes.

9. The use of any one of claims 1 to 8, wherein said pharmaceutical composition is suitable for administration to a ventricle in the central nervous system of a mammal.

10. The use of claim 9, wherein said administration is to be carried out by an osmotic pump.

11. The use of any one of claims 1 to 9, wherein said pharmaceutical composition is contained within an osmotic pump adopted for delivery of said composition to a ventricle in the central nervous system of a mammal.

12. The use of any one of claims 9 to 11, wherein said ventricle is a lateral ventricle of a mammal's forebrain and/or is the central canal in the spinal cord of a mammal and/or is a CNS ventricular tissue containing quiescent stem cells.

13. The use of any one of claims 9 to 12, wherein said mammal is a primate.

14. The use of any one of claims 1 to 13, wherein said neurological injury is selected from the group consisting of stroke, head injury and cerebral palsy.

15. The use of any one of claims 1 to 13, wherein said neurological disorder is selected from the group consisting of epilepsy, depression and schizophrenia.

16. The use of any one of claims 1 to 13, wherein said neurological disorder is selected from the group consisting of neurodegenerative diseases, demyelinating disorders and hypomyelinating disorders.

17. The use of claim 16, wherein said neurodegenerative diseases comprise Parkinson's disease, Huntington's disease, Alzheimer's disease and/or Amyotrophic Lateral Sclerosis.

18. The use of claim 16, wherein said demyelinating disorders comprise multiple sclerosis.

19. A method of *in vitro* proliferation of neural precursor cells which comprises
culturing a precursor cell in a culture medium comprising one or more growth factors that induce multipotent neural stem cell proliferation which precursor cell is derived from tissue lining a CNS ventricle and has been obtained by
administering a combination of fibroblast growth factor and epidermal growth factor to a CNS ventricle of a living mammal,
removing said tissue from said mammal and dissociating said tissue to separate said precursor cell from said tissue.

20. The method of claim 19, wherein said ventricle is a lateral ventricle of said mammal's forebrain.

21. The use of any one of claims 1 to 18 or the method of claims 19 or 20, wherein said fibroblast growth factor is acidic fibroblast growth factor (aFGF) and/or basic fibroblast growth factor (bFGF).

## Patentansprüche

1. Verwendung einer Kombination umfassend epidermalen Wachstumsfaktor und Fibroblasten-Wachstumsfaktor für die Herstellung eines Arzneimittels zur Behandlung von neurologischen Krankheiten, Störungen und/oder Schäden.

2. Verwendung nach Anspruch 1, wobei das Arzneimittel zusätzlich andere Moleküle umfasst, die einen Wachstums-, Differenzierungs-, trophischen und/oder regulatorischen Effekt haben.

3. Verwendung nach Anspruch 2, wobei die Moleküle ausgewählt sind aus der Gruppe bestehend aus Activin, Amphiregulin, dem Bcl-2-Genprodukt, morphogenetischem Knochenprotein (BMP-2), vom Gehirn stammendem neurotrophem Faktor (BDNF), ciliärem neurotrophem Faktor (CNTF), EGF-ähnlichem Liganden, von der Glia stammendem neurotrophem Faktor (GDNF), Heparin-ähnlichen Molekülen, Insulin-ähnlichem Wachstumsfaktor (IGF-1), Interleukinen, "macrophage inflammatory proteins" (MIP-1α, MIP-1β und MIP-2), Nerven-Wachstumsfaktor (NGF), Thrombocyten-Wachstumsfaktor (PDGF), Retinsäure, Thyroliberin (TRH), transformierenden Wachstumsfaktoren alpha und beta (TGFα und TGFβ), und Tumornecrosefaktor alpha (TNFα).

4. Verwendung nach einem der Ansprüche 1 bis 3, wobei das Arzneimittel zusätzlich Heparansulfat umfasst.

5. Verwendung nach einem der Ansprüche 1 bis 4, wobei das Arzneimittel zusätzlich genetisches Material zur genetischen Modifikation einer neuralen Vorläuferzelle umfasst.

6. Verwendung nach Anspruch 5, wobei sich das genetische Material in einem viralen Konstrukt befindet.

7. Verwendung nach Anspruch 6, wobei das virale Konstrukt ein retrovirales Konstrukt ist.

8. Verwendung nach einem der Ansprüche 5 bis 7, wobei das genetische Material neurologische Mittel codiert, ausgewählt aus der Gruppe bestehend aus Wachstumsfaktoren, Wachstumsfaktor-Rezeptoren, Neurotransmittern, Neurotransmitter-Rezeptoren, Neuropeptiden, Wachstumsfaktorsynthetisierenden Enzymen und Neurotransmitter-synthetisierenden Enzymen.

9. Verwendung nach einem der Ansprüche 1 bis 8, wobei das Arzneimittel zur Verabreichung in ein Ventrikel im zentralen Nervensystem eines Säugetieres geeignet ist.

10. Verwendung nach Anspruch 9, wobei die Verabreichung durch eine osmotische Pumpe ausgeführt wird.

11. Verwendung nach einem der Ansprüche 1 bis 9, wobei das Arzneimittel sich in einer osmotischen Pumpe befindet, welche zur Verabreichung des Arzneimittels in ein Ventrikel im zentralen Nervensystem eines Säugetieres ausgerichtet ist.

12. Verwendung nach einem der Ansprüche 9 bis 11, wobei das Ventrikel ein laterales Ventrikel eines Säugetiervorderhirns ist und/oder der Zentralkanal im Rückenmark eines Säugetiers ist und/oder ein ZNS-Ventrikulärgewebe ist, welches ruhende Stammzellen enthält.

13. Verwendung nach einem der Ansprüche 9 bis 12, wobei das Säugetier ein Primat ist.

14. Verwendung nach einem der Ansprüche 1 bis 13, wobei die neurologische Störung ausgewählt ist aus der Gruppe bestehend aus Schlaganfall, Kopfverletzung und zerebraler Lähmung.

15. Verwendung nach einem der Ansprüche 1 bis 13, wobei die neurologische Störung ausgewählt ist aus der Gruppe bestehend aus Epilepsie, Depression und Schizophrenie.

16. Verwendung nach einem der Ansprüche 1 bis 13, wobei die neurologische Störung ausgewählt ist aus der Gruppe bestehend aus neurodegenerativen Krankheiten, demyelinierenden Schäden und hypomyelinierenden Schäden.

17. Verwendung nach Anspruch 16, wobei die neurodegenerativen Krankheiten Parkinson-Krankheit, Chorea Huntington, Alzheimer-Krankheit und/oder amyotrophe Lateralsklerose umfassen.

18. Verwendung nach Anspruch 16, wobei die demyelinierenden Krankheiten Multiple Sklerose umfassen.

19. Verfahren zur *in vitro*-Proliferation neuronaler Vorläuferzellen, umfassend das Kultivieren einer Vorläuferzelle in einem Kulturmedium, das ein oder mehrere Wachstumsfaktoren umfasst, welche die Proliferation multipotenter neuronaler Stammzellen induzieren, wobei die Vorläuferzelle von Gewebe stammt, welches ein ZNS-Ventrikel auskleidet und erhalten wurde durch Verabreichung einer Kombination aus Fibroblasten-Wachstumsfaktor und epidermalem Wachstumsfaktor in ein ZNS-Ventrikel eines lebenden Säugetieres, Entfernen des Gewebes aus dem Säugetier und Dissoziieren des Gewebes, um die Vorläuferzelle vom Gewebe zu trennen.

20. Verfahren nach Anspruch 19, wobei das Ventrikel ein laterales Ventrikel des Säugetiervorderhirns ist.

21. Verwendung nach einem der Ansprüche 1 bis 18 oder Verfahren nach Anspruch 19 oder 20, wobei der Fibroblasten-Wachstumsfaktor ein saurer Fibroblasten-Wachstumsfaktor (aFGF) und/oder ein basischer Fibroblasten-Wachstumsfaktor (bFGF) ist.

## Revendications

1. Utilisation d'une combinaison comprenant le facteur de croissance épidermique et le facteur de croissance des fibroblastes pour la production d'une composition pharmaceutique pour le traitement des maladies, des troubles et/ou des lésions neurologiques.

2. Utilisation selon la revendication 1, dans laquelle ladite composition pharmaceutique contient de manière additionnelle d'autres molécules ayant un effet sur la croissance, sur la différenciation, un effet trophique et/ou régulateur.

3. Utilisation selon la revendication 2, dans laquelle lesdites molécules sont choisies dans le groupe constitué par l'activine, l'amphiréguline, le produit du gène Bcl-2, la protéine morphogénique osseuse (BMP-2), le facteur neurotrophique dérivé du cerveau (BDNF), le facteur neurotrophique ciliaire (CNTF), le ligand de type EGF, le facteur neurotrophique d'origine gliale (GDNF), les molécules du type héparine, le facteur de croissance de type insuline (IGF-1), les interleukines, les protéines inflammatoires des macrophages (MIP-1α, MIP-1β et MIP-2), le facteur de croissance nerveux (NGF), le facteur de croissance d'origine plaquettaire (PDGF), l'acide rétinoïque, l'hormone de libération de la thyréotropine (TRH), les facteurs de croissance transformants alpha et beta (TGFα, TGFβ), et le facteur de nécrose tumorale alpha (TNFα).

4. Utilisation selon l'une quelconque des revendications 1 à 3, dans laquelle ladite composition pharmaceutique comprend de manière additionnelle l'héparane sulfate.

5. Utilisation selon l'une quelconque des revendications 1 à 4, dans laquelle ladite composition pharmaceutique comprend de manière additionnelle du matériel génétique pour la modification génétique des cellules précurseurs neurales.

6. Utilisation selon la revendication 5, dans laquelle ledit matériel génétique est contenu dans une construction virale.

7. Utilisation selon la revendication 6, dans laquelle ladite construction virale est une construction rétrovirale.

8. Utilisation selon l'une quelconque des revendications 5 à 7, dans laquelle ledit matériel génétique code un agent neurologique choisi dans le groupe constitué par les facteurs de croissance, les récepteurs des facteurs de croissance, les neurotransmetteurs, les récepteurs des neurotransmetteurs, les neuropeptides, les enzymes synthétisant les facteurs de croissance et les enzymes synthétisant les neurotransmetteurs.

9. Utilisation selon l'une quelconque des revendications 1 à 8, dans laquelle ladite composition pharmaceutique convient pour l'administration dans un ventricule dans le système nerveux central d'un mammifère.

10. Utilisation selon la revendication 9, dans laquelle ladite administration est réalisée par une pompe osmotique.

11. Utilisation selon l'une quelconque des revendications 1 à 9, dans laquelle ladite composition pharmaceutique est contenue dans une pompe osmotique adoptée pour l'administration de ladite composition dans un ventricule du système nerveux central d'un mammifère.

12. Utilisation selon l'une quelconque des revendications 9 à 11, dans laquelle ledit ventricule est un ventricule latéral d'un cerveau antérieur de mammifère et/ou est le canal central de la moelle épinière d'un mammifère et/ou est un tissu ventriculaire du SNC contenant des cellules souches quiescentes.

13. Utilisation selon l'une quelconque des revendications 9 à 12, dans laquelle ledit mammifère est un primate.

14. Utilisation selon l'une quelconque des revendications 1 à 13, dans laquelle ladite lésion neurologique est choisie dans le groupe constitué par les accidents vasculaires cérébraux, les traumatismes crâniens et les paralysies cérébrales.

15. Utilisation selon l'une quelconque des revendications 1 à 13, dans laquelle ledit trouble neurologique est choisi dans le groupe constitué par l'épilepsie, la dépression et la schizophrénie.

16. Utilisation selon l'une quelconque des revendications 1 à 13, dans laquelle ledit trouble neurologique est choisi dans le groupe constitué par les maladies neurodégénératives, les troubles de la démyélinisation et les troubles de l'hypomyélinisation.

17. Utilisation selon la revendication 16, dans laquelle lesdits troubles neurodégénératifs comprennent la maladie de Parkinson, la chorée de Huntington, la maladie d'Alzheimer et/ou la sclérose latérale amyotrophique.

18. Utilisation selon la revendication 16, dans laquelle lesdits troubles de la démyélinisation comprennent la sclérose en plaques.

19. Méthode de prolifération *in vitro* de cellules précurseurs neurales qui comprend la culture d'une cellule précurseur dans un milieu de culture comprenant un ou plusieurs facteurs de croissance qui indui(sen)t la prolifération des cellules souches neurales multipotentes, laquelle cellule précurseur provient du tissu bordant un ventricule du SNC et a été obtenue par l'administration d'une combinaison de facteur de croissance fibroblastique et de facteur de croissance épidermique à un ventricule du SNC d'un mammifère vivant, le prélèvement dudit tissu dudit mammifère et la dissociation dudit tissu pour séparer ladite cellule précurseur dudit tissu.

20. Méthode selon la revendication 19, dans laquelle ledit ventricule est un ventricule latéral dudit cerveau antérieur de mammifère.

21. Utilisation selon l'une quelconque des revendications 1 à 18 ou la méthode selon les revendications 19 ou 20, dans laquelle ledit facteur de croissance des fibroblastes est le facteur de croissance acide des fibroblastes (aFGF) et/ou le facteur de croissance basique des fibroblastes (bFGF).
